# FASCICULE DE BREVET EUROPEEN

(11) **EP 2 181 666 B1**
(45) Date de publication et mention de la délivrance du brevet: **17.10.2018**
(21) Numéro de dépôt: 09174380.7
(22) Date de dépôt: 28.10.2009
(51) Int. Cl.: A61L 31/04, A61L 31/14, A61F 2/00

(54) **Membrane chirurgicale antiadhérence**
Antihaft-chirurgie Membran
Non-Adhesion surgery membrane

(30) Priorité: 30.10.2008 FR 0806055
(43) Date de publication de la demande: 05.05.2010
(73) Titulaire: Les Laboratoires Brothier, 92000 Nanterre (FR)
(72) Inventeur: Barikosky, Michel, 75007 Paris (FR); Girardiere, Christian, 75116 Paris (FR); Lack, Stéphane, 49070 Beaucouzé (FR)
(74) Mandataire: Gevers & Orès

(56) Documents cités:
- WO-A-99/45860
- DE-A1-102004 051 487
- FR-A- 2 857 851
- US-A1- 2008 086 216

## Description

Le domaine de l' invention est celui des adhérences.

Les adhérences sont des bandes fibreuses qui relient des surfaces tissulaires normalement isolées. La formation d'adhérences est une conséquence possible de toute intervention chirurgicale, puisqu'elle s'inscrit dans le cadre du processus cicatriciel.

Des adhérences se développent chez la plupart des patients ayant subi une intervention chirurgicale à l'abdomen ou dans la région pelvienne, comme une cure de hernie, ou une intervention gynécologique ou colorectale. Ces adhérences sont également inhérentes à la chirurgie tendineuse, cardiaque.

Les adhérences peuvent être à l'origine de graves complications : l'occlusion de l'intestin grêle, la stérilité chez la femme, des douleurs chroniques, des difficultés lors d'interventions subséquentes, etc. Près de trois cas d'occlusion intestinale sur quatre et de un cas de douleur pelvienne chronique sur cinq, voire sur deux, sont imputables à des adhérences post-chirurgicales. Il n'est pas rare que de nouvelles adhérences se forment par suite d'une intervention chirurgicale ayant précisément pour but d'éliminer les anciennes, ce qui a pour effet, outre une nouvelle opération, de prolonger le temps de guérison et d'augmenter le risque, le coût et la complexité de l'intervention.

L'invention concerne une membrane chirurgicale antiadhérence, qu'on utilise donc notamment en chirurgie digestive pour l'intercaler entre l'intestin et la paroi abdominale. De façon générale, on en utilise pour empêcher l'adhérence mutuelle des tissus, par exemple encore ceux d'un tendon et de la peau.

Les membranes bioadhésives antiadhérence peuvent être obtenues par évaporation de l'eau d'une solution d'eau et de quelques pourcents d'un ou plusieurs polymères. Les membranes sont pour ainsi dire plastifiées. Comme polymère, on peut considérer un ester d'alginate, comme, par exemple, l'alginate de polyéthylène glycol, additionné de poloxamer et de cuivre.

Les membranes actuellement disponibles sur le marché manquent de souplesse et l'invention de la présente demande vise à augmenter cette souplesse.

Ainsi, l'invention concerne une membrane chirurgicale antiadhérence en matériau polymère, caractérisée par le fait que l'une de ses deux faces au moins présente des reliefs d'assouplissement et de résorbabilité.

Les reliefs sont dits d'assouplissement car ils ont été obtenus au cours de la fabrication, par formage qui a apporté de l'anisotropie et ainsi augmenté la souplesse de la membrane.

Les reliefs sont dits de résorbabilité car, en cours d'usage, grâce aux canaux qu'ils forment entre eux, ils peuvent retenir le liquide biologique qui, incidemment, peut faire fonction de lubrifiant.

On remarquera que les reliefs permettent de diminuer la surface de contact de la membrane, grâce à quoi on peut la déplacer plus facilement sur son site, pour la positionner correctement. A cet égard, les reliefs peuvent être également qualifiés de relief de contact.

On remarquera de plus que les reliefs peuvent favoriser la résorption de la membrane de l'invention, sa disparition au bout d'un certain temps, après la cicatrisation constituant une de ses qualités.

Le matériau de la membrane de l'invention peut également comprendre un agent réticulant, un adjuvant, un principe actif.

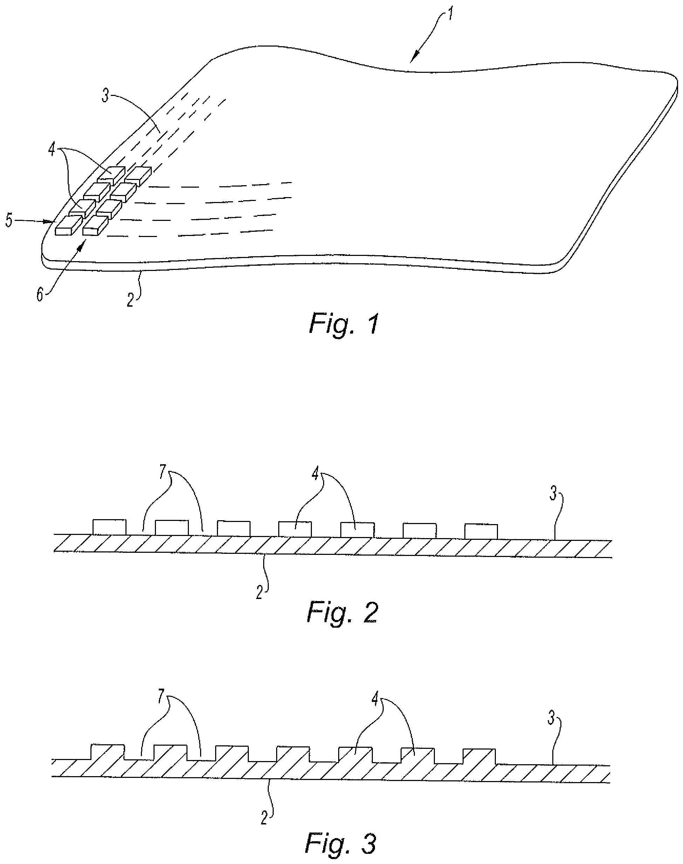

L'invention sera mieux comprise à l'aide de la description suivante d'une forme de réalisation intéressante de la membrane de l'invention, en référence au dessin en annexe, sur lequel
- la figure 1 est une vue en perspective du dessus de la membrane ;
- la figure 2 est une vue en coupe de la membrane, à travers un canal de résorbabilité et
- la figure 3 est une vue en coupe de la membrane à travers une rangée de plots bordant un canal de résorbabilité.

Pour former la membrane qui va être décrite, on commence par créer une partie de moule avec un fond pourvu de petits alvéoles ouverts, sensiblement parallélépipédiques, régulièrement répartis à la surface de ce fond, suivant ici un agencement matriciel de rangées et de colonnes. Il peut s'agir d'un conformateur de type poinçon matrice.

On forme une solution d'eau et d'un polymère. On la verse dans le moule et on laisse l'eau s'évaporer jusqu'à l'obtention d'une membrane plastifiée 1 présentant, d'un côté, une face plane 2 et, de l'autre côté, une face texturée, ou tourmentée, 3, avec des reliefs, ou protubérances, en forme de petits plots parallélépipédiques 4, correspondant aux alvéoles du moule. Les plots sont répartis sur la face 3 de façon matricielle en rangées 5 et colonnes 6 bordant des canaux de résorbabilité 7 ici orthogonaux entre eux.

Comme solution de base pour la formation de la membrane, on a considéré ici une solution d'alginate de propylène glycol (ester d'alginate) réticulé, additionnée, ici, d'un produit de la marque protégée Lutrol®, de glycérol (adjuvant) et de cuivre, comme ion de réticulation. La proportion de polymère, dans l'espèce considérée, était de 2% en volume. Une proportion de 0,5% à 10% de polymère est convenable. Cette plage se justifie pour éviter que la solution soit trop visqueuse ou trop aqueuse.

Comme agent de réticulation pour les esters d'alginate, on peut considérer, de façon générale, tous ions multivalents, comme précisément le cuivre. Comme principe actif, on peut considérer une vitamine, un agent anti-inflammatoire, un antioxydant.

L'addition d'un complexe vitaminique (principe actif) permet d'améliorer la réparation des tissus lésés lors de l'acte chirurgical.

On obtient ainsi des reliefs d'assouplissement, de résorbabilité et de contact.

Grâce à la formation des reliefs, la membrane ainsi obtenue est pourvue d'une grande souplesse, elle offre une intéressante capacité de résorption et un repositionnement aisé. Elle peut être conditionnée en rouleau.

On a décrit une membrane antiadhérence pourvue de reliefs sur l'une de ses deux faces seulement. On pourrait envisager une membrane texturée sur ses deux faces.

Dans le cas ou la membrane n'est texturée que sur l'une de ses faces, l'autre face pourrait être adhésive,

Naturellement, la membrane de l'invention pourrait être associée à un renfort souple pour utilisation dans certaines applications particulières.

On a décrit une membrane à reliefs parallèlèpipédiques. Naturellement, il ne peut s'agir d'une caractéristique limitative. Les reliefs pourraient être tronconiques, arrondis, cylindriques. L'agencement des reliefs peut également ne pas être aussi régulier qu'un agencement matriciel.

## Revendications

1. Membrane chirurgicale antiadhérence en matériau polymère, l'une (3) de ses deux faces au moins présentant des reliefs d'assouplissement et de résorbabilité (4), **caractérisée en ce qu'**elle est formée par évaporation d'une solution d'eau et de 0,5 à 10% en volume de polymère.

2. Membrane selon la revendication 1, dans laquelle le dit matériau comporte un dérivé d'ester d'alginate.

3. Membrane selon la revendication 1 ou 2, qui a été formée par évaporation de la solution dans un moule comportant des alvéoles correspondant aux reliefs (4) de la membrane.

## Patentansprüche

1. Chirurgische Antihaftmembran aus Polymermaterial, wobei eine (3) ihrer beiden Seiten mindestens weichmachende und resorbierbare Reliefs (4) aufweist, **dadurch gekennzeichnet, dass** sie durch Verdampfen einer Wasserlösung und 0,5 bis 10 Vol.-% Polymer gebildet ist.

2. Membran nach Anspruch 1, wobei das Material ein Alginatesterderivat enthält.

3. Membran nach Anspruch 1 oder 2, die durch Verdampfen der Lösung in einer Form, die Zellen enthält, die den Reliefs (4) der Membran entsprechen, gebildet worden ist.

## Claims

1. Anti-adherence surgical membrane made of polymeric material, at least one (3) of its two faces having softening and resorbing reliefs (4), **characterised in that** it is formed by evaporation of a water solution and of 0.5 to 10 % by volume of polymer.

2. Membrane according to claim 1, wherein said material features an alginate ester derivative.

3. Membrane according to claim 1 or 2, formed by evaporation of the solution in a mould comprising cavities corresponding to the reliefs (4) of the membrane.
